# DEMANDE DE BREVET EUROPEEN

(11) **EP 0 687 448 A1**
(43) Date de publication de la demande: **20.12.1995**
(21) Numéro de dépôt: 95401387.6
(22) Date de dépôt: 14.06.1995
(51) Int. Cl.: A61B 17/14, A61B 17/16, A61F 2/38

(54) **Prothèse fémoro-patellaire et dispositif ancillaire pour former une empreinte trochléenne de réception de cette prothèse**

(30) Priorité: 16.06.1994 FR 9407392
(71) Demandeur: Labourdette, Patrick Marie Croly, F-59300 Valenciennes (FR)
(72) Inventeur: Labourdette, Patrick Marie Croly, F-59300 Valenciennes (FR)
(74) Mandataire: Beauchamps, Georges

(57) **Abrégé**

La présente invention concerne un dispositif ancillaire pour la résection partielle de l'extrémité distale du fémur.

Le dispositif de l'invention comporte un guide intramédullaire (3), un support (17) monté amovible sur le guide, une plaque allongée (6) pour le positionnement par rapport aux extrémités postérieures des condyles (7), un guide de coupe (26) pour recevoir une rape cylindrique (1) formant outil de coupe, le guide de coupe comportant des moyens de guidage en translation (33a, 35) de la rape suivant deux plans sécants et le support comportant des moyens (23, 27) pour déplacer le guide de coupe.

L'invention s'applique notamment à la formation d'une empreinte en creux dans l'extrémité distale du fémur pour la réception d'une prothèse fémoro-patellaire.

## Description

La présente invention concerne une prothèse fémoro-patellaire ou trochléenne et un dispositif ancillaire pour la résection partielle de la corticale antérieure distale, du fémur pour former une empreinte en creux de réception de cette prothèse fémoro-patellaire.

On observe dans certains cas, par exemple dans l'arthrose et dans l'arthrite rhumatoïde, une déformation ou une détérioration du genou telle qu'il est nécessaire d'implanter une prothèse articulaire.

Lorsque cette détérioration est limitée à la trochlée de l'extrémité distale du fémur, à savoir sa partie médiane, et si les cartilages articulaires des condyles sont en bon état, au lieu d'effectuer une arthroplastie totale du genou, on préfére remplacer partiellement l'articulation en effectuant une résection de la surface abîmée du fémur pour ménager une empreinte de réception d'une prothèse trochléenne sur laquelle vient en appui un bouton rotulien implanté sur la rotule.

On effectue généralement, pour restituer la pente de la trochlée externe, une coupe de la corticale antérieure distale du fémur suivant deux plans sécants, à savoir un plan tangent à la face antérieure de la diaphyse fémorale et un plan incliné situé à 1 ' extrémité du fémur, pour former deux surfaces d'appui constituant l'empreinte sur lesquelles vient s'appuyer la face postérieure d'une prothèse fémoro-patellaire.

L'opération consistant à couper une partie de l'extrémité distale du fémur et à implanter une prothèse fémoro-patellaire est très délicate, car le succès de l'opération dépend essentiellement de la précision des coupes osseuses et du positionnement anatomique correct de la prothèse sur l'extrémité fémorale.

En effet, l'implantation d'une prothèse fémoro-patellaire impose que la résection de l'os soit strictement limitée à la partie médiane et frontale de l'extrémité fémorale et que l'intégrité des condyles et des cartilages articulaires qui les recouvrent soit préservée.

D'autre part, l'ancrage des prothèses fémoro-patellaires connues sur l'extrémité distale du fémur s'effectue généralement au moyen de plots longs qui s'engagent profondément dans l'os. L'utilisation de ces longs clous est rendue nécessaire par l'imprécision de la résection de l'os fémoral et donc par la mauvaise qualité de l'empreinte de réception de la prothèse.

Cet ancrage profond des plots dans l'os fémoral a pour inconvénient majeur qu'il augmente le risque de nécrose de l'os, ce qui peut entraîner, dans un délai relativement court, le remplacement de cette prothèse fémoro-patellaire par une prothèse totale du genou.

En outre, l'imprécision des coupes osseuses et donc l'adaptation imparfaite de la prothèse dans son empreinte diminue la stabilité primaire de la prothèse sur l'extrémité fémorale.

On ne connaît pas actuellement d'instrumentation ancillaire permettant d'effectuer ce type d'opération de manière satisfaisante et la résection de l'os pour la pose d'une prothèse fémoro-patellaire s'effectue généralement au moyen d'un gabarit de perçage présentant la même forme que la prothèse trochléenne à implanter et que le chirurgien positionne à l'aide de son savoir-faire.

Il existe cependant des dispositifs ancillaires permettant d'effectuer une arthroplastie totale du genou pour la mise en place d'une prothèse totale.

On connaît déjà un dispositif ancillaire permettant de remodeler intégralement l'extrémité fémorale en réalisant une pluralité de faces planes dans l'os fémoral que vient coiffer la prothèse totale.

Un tel dispositif ancillaire connu comprend par exemple :
- un guide d'alignement intramédullaire, tel qu'une tige, que l'on introduit dans l'extrémité distale du fémur suivant l'axe anatomique du fémur,
- un support monté amovible sur le guide intramédullaire et comprenant des moyens de positionnement par rapport aux repères anatomiques du fémur, à savoir une plaque allongée venant s'appuyer sur l'extrémité distale des condyles le long de la ligne bicondylienne et un palpeur venant en contact de la surface corticale antérieure du fémur,
- une pluralité de guides de coupe constitués chacun d'une pièce massive métallique à travers laquelle est ménagée au moins une fente pour le passage de la lame d'une scie, à savoir par exemple un guide de coupe antérieure des condyles fémoraux, un guide de coupe du fémur distal, un guide de coupe des condyles fémoraux antérieur et postérieur, un guide de recoupe du fémur distal, et un guide de coupe de l'échancrure intercondylienne et des chanfreins, et
- une pluralité de guides de mesure, tels qu'un guide de mesure de la dimension antéropostérieure du fémur distal et un guide de mesure des écarts de flexion et d'extension du genou.

Ce dispositif ancillaire connu présente de nombreux inconvénients, notamment les guides de coupe ne permettent pas de contrôler la direction de coupe de la lame de scie introduite dans la fente de ces guides.

En outre, ce dispositif ne peut convenir pour effectuer une résection de la zone trochléenne de l'articulation du genou, car ce dispositif connu ne permet pas de préserver la zone saine de l'articulation.

Ce dispositif connu a également pour inconvénient que la fixation du support sur l'os s'effectue à l'aide de clous qui sont introduits selon l'axe longitudinal du guide intramédullaire et enfoncés dans les zones des condyles revêtues de cartilage articulaire et présumé fin.

Pour la mise en place d'une prothèse totale, ces clous ne sont pas gênants car les condyles seront supprimés mais, dans le cas d'une prothèse fémoro-patellaire, il est nécessaire de préserver la partie saine de l'articulation notamment les cartilages qui recouvrent les condyles dans la zone frontale.

Ce dispositif connu est constitué de nombreuses pièces et utilise de nombreux outils, ce qui rend son utilisation complexe et coûteuse pour une simple résection partielle de l'extrémité distale du fémur.

La présente invention a donc pour but d'éliminer les inconvénients précités et de proposer un dispositif ancillaire permettant d'effectuer une résection précise de l'os fémoral, de préserver les parties saines de l'articulation et qui soit de structure simple et économique.

A cet effet, la présente invention a pour objet un dispositif ancillaire pour la résection partielle de l'extrémité distale du fémur, comportant :
- un guide d'alignement intramédullaire, tel qu'une tige, à introduire dans l'extrémité distale du fémur suivant l'axe anatomique de celui-ci,
- un support monté, de préférence amovible, sur le guide intramédullaire et supportant des moyens de positionnement par rapport aux repères anatomiques du fémur, à savoir par exemple la ligne bicondylienne, l'extrémité distale des condyles et la surface corticale antérieure du fémur située juste au-dessus de la trochlée,
- un guide de coupe pour recevoir au moins un outil de coupe,
   caractérisé en ce que l'outil de coupe est un organe cylindrique dont la surface de révolution est apte à user ou à raper la zone trochléenne de l'extrémité fémorale sous l'action d'un arbre moteur solidaire en rotation dudit organe, et que
- le guide de coupe précité comporte des moyens de guidage en translation de l'outil de coupe suivant deux plans sécants, l'un tangent à la corticale antérieure et l'autre incliné sensiblement parallèlement à la zone intracondylienne.

Le guide de coupe peut être monté avantageusement amovible sur le support précité qui comporte en outre des moyens de déplacement du guide de coupe.

Selon une caractéristique particulière de l'invention, les moyens de déplacement précités sont formés de deux liaisons glissières orientées respectivement perpendiculairement à la corticale antérieure et parallèlement au guide intramédullaire.

Les moyens de positionnement précités comprennent par exemple un moyen de butée formant palpeur qui fait saillie vers le bas de l'extrémité libre avant du guide de coupe, la taille du palpeur étant prédéterminée en fonction du rayon de l'outil de coupe utilisé pour régler automatiquement la distance séparant les moyens de guidage de la corticale antérieure, par aboutement du palpeur sur celle-ci.

Selon une autre caractéristique de l'invention, un tronçon du guide intramédullaire est cintré et positionné entre l'extrémité fémorale distale et le support précité pour laisser un espace suffisant pour permettre l'évolution de l'outil de coupe lorsque ce dernier effectue une coupe du fémur dans la zone intercondylienne inclinée.

Avantageusement, les moyens de positionnement comprennent en outre une plaque allongée destinée à venir s'appuyer sur l'extrémité distale des condyles le long de la ligne bicondylienne, et des clous de fixation traversant une extrémité longitudinale de ladite plaque pour venir se planter dans la zone des condyles non recouverte de cartilage, ladite plaque étant constituée de deux éléments dont l'un est coulissant par rapport à l'autre dans la direction longitudinale de la plaque pour régler sa longueur en fonction de l'écartement des condyles, et ladite plaque étant reliée audit support par un bras articulé autour d'un axe sensiblement perpendiculaire à la corticale antérieure.

Selon encore une autre caractéristique de l'invention, les moyens de guidage se présentent sous la forme d'un cadre rectangulaire dont les deux longerons latéraux ont pour fonction de supporter l'arbre moteur de part et d'autre de l'organe cylindrique formant outil de coupe, chaque longeron présentant une forme sensiblement en chevron qui définit une surface de guidage principale parallèle à la corticale antérieure et se prolongeant par un logement de guidage en forme de U incliné, dans lequel l'arbre moteur est reçu pour réaliser la surface d'appui inclinée dans le fémur.

Le guide de coupe peut comporter éventuellement un étrier surmontant le cadre précité, servant de butée supérieure dans le cas d'un débattement vertical de l'outil de coupe et ouvert vers l'avant pour permettre l'introduction et le retrait rapides de l'outil de coupe dans le guide de coupe.

Le guide de coupe peut également comporter une paire de cales de forme complémentaire aux logements de guidage en U incliné et comportant une surface supérieure plane définissant un plan unique avec la surface de guidage principale des longerons latéraux lorsque les cales sont reçues dans les logements précités.

Dans un mode de réalisation particulier de l'invention, le support présente une forme générale en T qui est constituée de deux équerres qui coulissent l'une par rapport à l'autre dans la direction de la jambe du T pour constituer la liaison glissière orientée parallèlement au guide intramédullaire.

Le dispositif ancillaire de l'invention peut comporter un outil de coupe en forme de rape ou de roue dentée dont les dents sont radialement inclinées pour venir creuser une rainure allongée dans la trochlée fémorale.

Selon une autre caractéristique de l'invention, le dispositif ancillaire comprend un gabarit de réglage de la position du guide de coupe à une distance adaptée du fémur pour former la surface d'appui inclinée de l'empreinte trochléenne, ce gabarit présentant la forme d'un cylindre lisse dont le rayon est égal au rayon de l'organe cylindrique précité minoré de la profondeur maximale de l'empreinte à former dans la trochlée.

On peut avantageusement prévoir une bague en plastique ou analogue formée d'une partie de diamètre supérieur et d'une partie de diamètre inférieur qui sont séparées par un épaulement radial, ladite bague étant destinée à s'emmancher sur l'arbre moteur pour assurer un bon centrage de l'outil de coupe sur le guide de coupe par l'intermédiaire de la partie de diamètre supérieur, la partie de diamètre inférieur servant à caler l'arbre moteur dans le guide de coupe.

La bague précitée peut appartenir à une gamme de modules de dimensions prédéterminées en fonction des outils de coupe utilisés pour former différentes tailles d'empreinte trochléenne.

La présente invention vise également une prothèse fémoro-patellaire à implanter dans une empreinte trochléenne réalisée de préférence avec le dispositif ancillaire de l'invention, caractérisée en ce qu'elle comprend au moins une nervure allongée en saillie de sa face postérieure, de préférence au centre de celle-ci.

Selon une caractéristique particulière de l'invention, la prothèse fémoro-patellaire comprend au moins un ergot en saillie de sa face postérieure.

L'invention sera mieux comprise et d'autres buts, caractéristiques, détails et avantages de celle-ci apparaîtront plus clairement dans la description explicative qui va suivre de plusieurs modes de réalisation particuliers, actuellement préférés de l'invention, donnés uniquement à titre d'exemples illustratifs et non limitatifs, en référence aux dessins schématiques annexés, dans lesquels :

La figure 1 est une vue schématique et en perspective d'un premier mode de réalisation du dispositif ancillaire de l'invention.

La figure 2 est une vue schématique et en perspective de l'extrémité fémorale inférieure dans laquelle est réalisée une empreinte de réception d'une prothèse fémoro-patellaire de l'invention.

La figure 3 est une vue schématique et en perspective d'une prothèse fémoro-patellaire de l'invention.

La figure 4 est une vue latérale suivant la flèche XII de la prothèse fémoro-patellaire de la figure 3.

La figure 5 est une vue schématique et en perspective représentant l'étape de forage de l'extrémité distale du fémur suivant son axe anatomique.

La figure 6 est une vue schématique et en perspective d'un guide intramédulaire du dispositif ancillaire de invention, implanté dans le canal intramédullaire du fémur de la figure 5.

La figure 7 est une vue schématique et en perspective d'un premier outil de coupe du dispositif ancillaire de l'invention.

La figure 8 est une vue schématique et en perspective d'un second outil de coupe du dispositif ancillaire de l'invention.

La figure 9 est une vue de dessus d'un support du dispositif ancillaire de l'invention supporté par le guide intramédullaire de la figure 6.

La figure 9A est une vue plane de l'élément coulissant de la plaque allongée montée sur le support de la figure 9.

La figure 9B est une vue en perspective de l'élément articulé associé à l'élément coulissant de la figure 9A.

La figure 10 est une vue de dessus d'une variante de réalisation du dispositif ancillaire de l'invention, après assemblage d'un guide de coupe sur le support illustré sur la figure 9.

La figure 11 est une vue en élévation latérale du dispositif ancillaire de la figure 10, associé à l'outil de coupe représenté sur la figure 7 pour former une surface d'appui principale dans la trochlée fémorale.

La figure 12 est une vue en élévation latérale du dispositif ancillaire de la figure 11, associé à un gabarit de réglage de la position du guide de coupe.

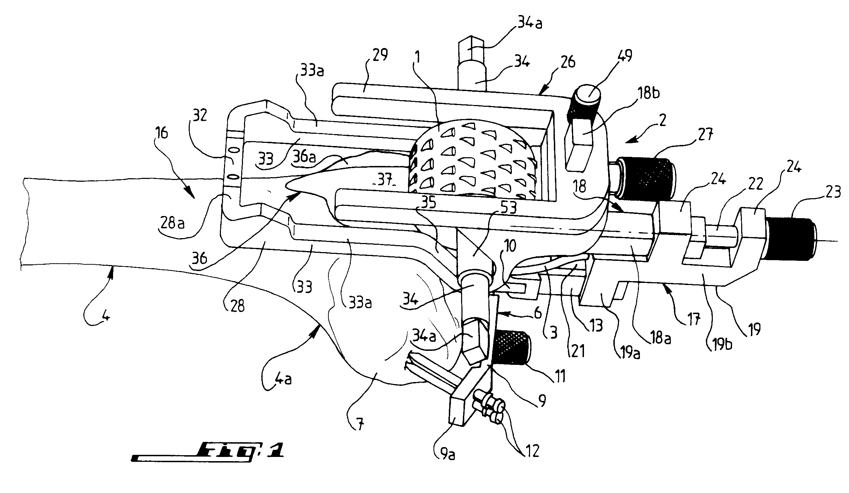

Suivant l'exemple de réalisation représenté sur la figure 1, le dispositif ancillaire de l'invention est constitué d'un outil de coupe formant rape 1 et d'un appareil 2 pour la résection de la trochlée fémorale.

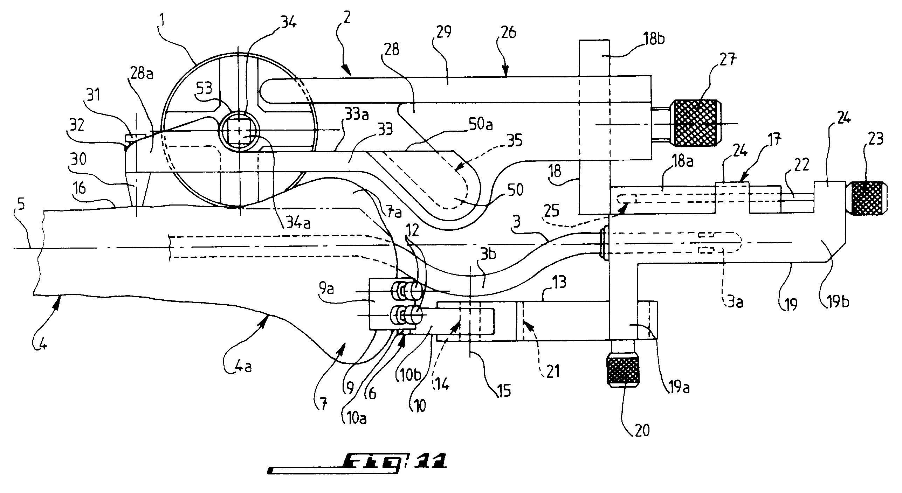

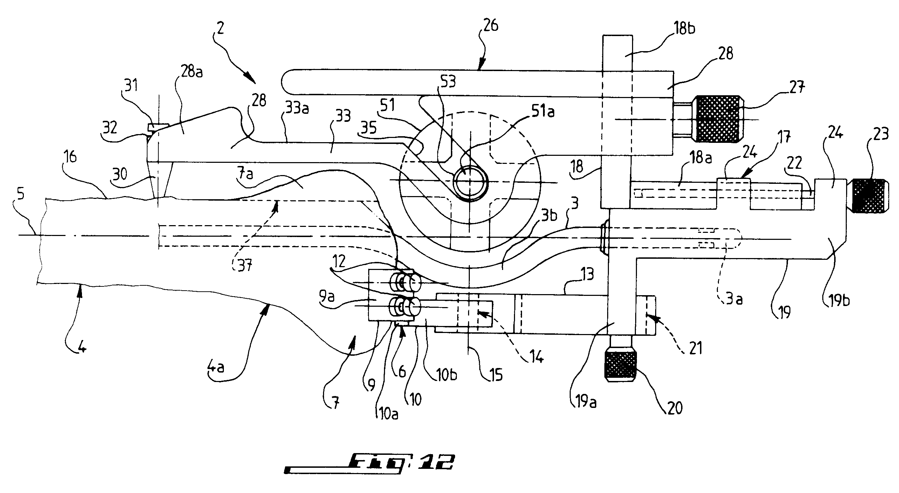

L'appareil 2 comprend :
- un guide d'alignement intramédullaire 3, par exemple une tige de 20 cm de longueur, à introduire dans l'extrémité distale 4a du fémur 4, suivant son axe anatomique 5 (voir figure 9),
- une plaque allongée 6 destinée à venir d'appuyer sur l'extrémité distale des condyles 7, le long de la ligne bicondylienne 8 (voir figure 9) et comprenant deux éléments 9 et 10 dont l'un 9 est coulissant par rapport à l'autre 10 dans la direction longitudinale de la plaque 6 pour régler sa longueur en fonction de l'écartement intercondylien, une mollette 11 servant à serrer les éléments 9 et 10 en position,
- des clous 12 traversant l'extrémité libre longitudinale 9a de l'élément coulissant 9 de ladite plaque 6 pour venir se planter latéralement dans les condyles 7,
- un bras 13 relié de manière articulée à l'élément 10 de la plaque 6 par l'intermédiaire d'une goupille 14 rotative autour d'un axe 15 (voir figure 11) perpendiculaire à la corticale antérieure 16 du fémur 4,
- un support 17 présentant une forme générale en T et constitué de deux équerres supérieure 18 et inférieure 19, de forme globalement en L, qui coulissent l'une par rapport à l'autre dans la direction de la jambe verticale du T, à savoir l'axe anatomique 5 du fémur,
- l'équerre inférieure 19 recevant à son extrémité inférieure 19a l'autre extrémité du bras articulé 13, une mollette 20 (voir figure 11) venant immobiliser le bras 13 par rapport à l'extrémité inférieure 19a à travers un trou oblong 21 ménagé à travers le bras 13,
- une extrémité 3a du guide intramédullaire 3 venant se loger dans le corps principal 19b de l'équerre inférieure 19 (voir figure 11),
- une liaison glissière à vis 22 actionnée par une mollette 23 et reliant de manière coulissante l'équerre supérieure 18 à l'équerre inférieure 19, ladite vis 22 et la branche horizontale 18a de l'équerre supérieure 18 traversant respectivement deux paliers 24 en forme de plots en saillie du corps principal 19b de l'équerre inférieure 19, ladite vis 22 venant se loger dans un taraudage 25 (figure 11) formé dans la branche horizontale 18a précitée,
- un guide de coupe 26 monté coulissant sur la branche verticale 18b de l'équerre supérieure 18 et immobilisé en position verticale par une mollette 27, ledit guide de coupe 26 comportant un cadre 28 surmonté d'un étrier de butée supérieure 29,
- un palpeur 30 (voir figure 11) qui fait saillie vers le bas de l'extrémité avant 28a du cadre 28 pour venir en butée contre le cortex métaphysaire antérieur 16 du fémur 4, des clous 31 traversant ledit palpeur 30 pour venir s'enfoncer dans la corticale antérieure 16 en prenant appui sur un lamage 32 ménagé à l'extrémité libre avant 28a du cadre 28, et
- le cadre rectangulaire 28 comportant deux longerons latéraux parallèles 33 servant à supporter un arbre moteur 34 de part et d'autre de la rape 1, chaque longeron 33 présentant une forme sensiblement en chevron définissant une surface principale de guidage 33a parallèle à la corticale antérieure 16 et qui se prolonge par un logement de guidage 35 en forme de U incliné dans lequel l'arbre moteur 34 est reçu.

L'élément coulissant 9, représenté plus clairement sur la figure 9A, comporte une lumière oblongue 9b pour recevoir la tige filetée de la molette 11 et régler longitudinalement la position de l'élément 9 par rapport à l'élément 10 de la plaque allongée 6, et un rebord latéral 9c ménagé en saillie du plan de l'élément coulissant 9 pour servir de butée et de guidage longitudinal sur l'élément 10.

L'élément articulé 10, représenté plus clairement sur la figure 9B, présente une forme générale en T dont les branches latérales 10a sont percées pour recevoir la tige filetée de la molette 11 à travers la lumière oblongue 9b et dont l'âme 10b est articulée à son extrémité libre autour de l'axe 15 précité.

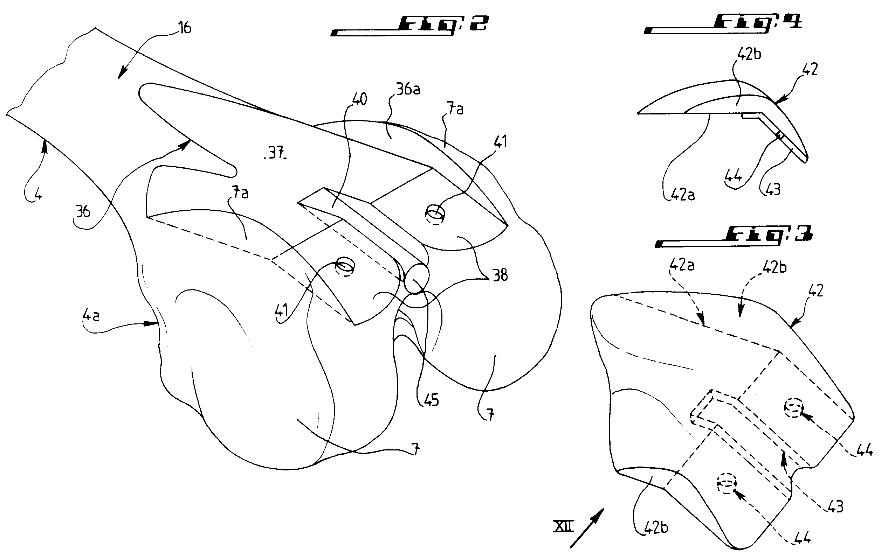

On a représenté sur les figures 1 et 2 l'empreinte trochléenne 36 réalisée dans l'extrémité fémorale 4a au moyen du dispositif ancillaire de l'invention.

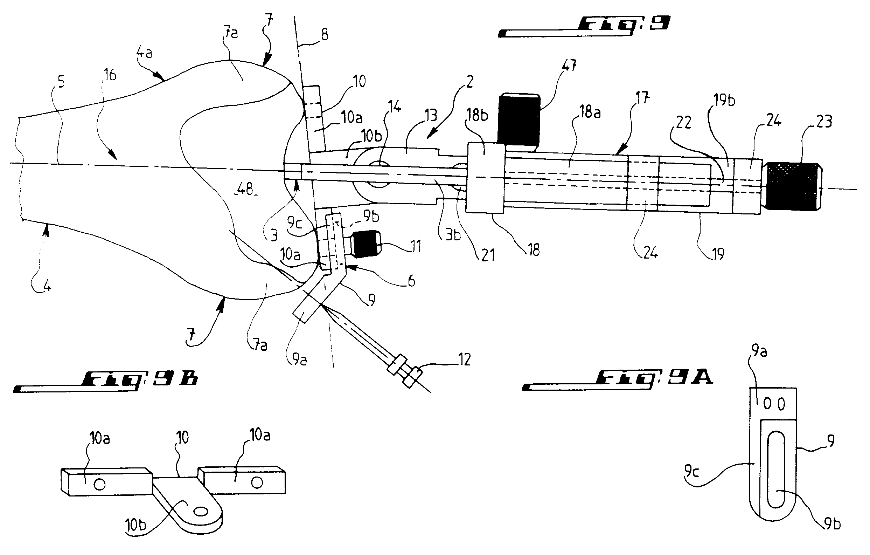

Cette empreinte trochléenne 36 est formée d'une surface d'appui principale 37 plane et tangente à la corticale antérieure 16 et se prolongeant longitudinalement par une surface d'appui inclinée 38 sensiblement parallèle à la zone intracondylienne 39 (voir figures 5 et 6) de la trochlée fémorale et d'une rainure médiane 40 ménagée plus profondement dans l'extrémité fémorale distale 4a.

La rainure 40 présente ici une inclinaison sensiblement identique à l'inclinaison de la surface d'appui inclinée 38, mais elle pourrait être réalisée en variante dans la surface d'appui principale 37 ou dans les deux surfaces d'appui 37 et 38.

On peut également prévoir deux orifices 41, ménagés par exemple dans la surface d'appui 38, mais ces orifices peuvent être d'un nombre quelconque et disposés dans un endroit quelconque de l'empreinte trochléenne 36.

Les surfaces d'appui 37 et 38 sont limitées à la partie médiane et frontale de l'extrémité fémorale et longitudinalement bordées sur leurs côtés par une surface 36a sensiblement perpendiculaire auxdites surfaces d'appui et préservant l'intégrité de l'extrémité antérieure 7a des condyles 7 et des cartilages qui les recouvrent.

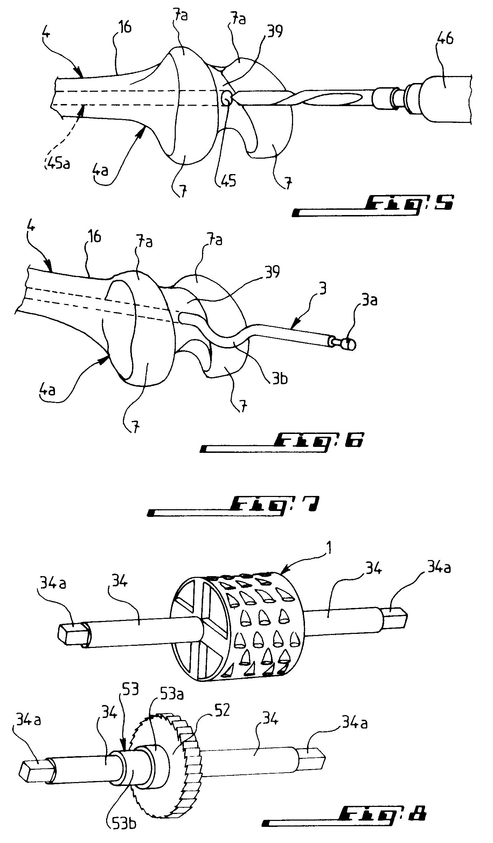

Les figures 3 et 4 représentent un exemple particulier de réalisation d'une prothèse fémoro-patellaire ou trochléenne 42 destinée à reconstituer la zone de l'articulation du genou recevant la rotule du genou (non représentée).

La prothèse 42 présente une face postérieure 42a et des faces latérales 42b de forme respectivement adaptée aux surfaces d'appui 37 et 38 et à la surface 36a de l'empreinte trochléenne 36, à partir de laquelle face postérieure 42a font saillie une nervure centrale 43 destinée à venir se loger dans la rainure 40 de l'empreinte 36 et deux ergots 44 destinés à venir se loger dans les orifices 41 précités, lesdites faces latérales 42b ayant une hauteur différente d'un côté à l'autre.

Bien entendu, la forme et la taille de la nervure 43 et des ergots 44 peuvent être modifiées sans sortir du cadre ni de l'esprit de la présente invention.

Par exemple, on pourrait prévoir plusieurs nervures s'adaptant dans plusieurs rainures de l'empreinte trochléenne.

On voit clairement sur les figures 1 et 2, que l'extrémité antérieure 7a des condyles 7 est préservée lors de la coupe du fémur et que seule la zone trochléenne est remplacée par une prothèse qui vient se loger dans l'empreinte ainsi formée.

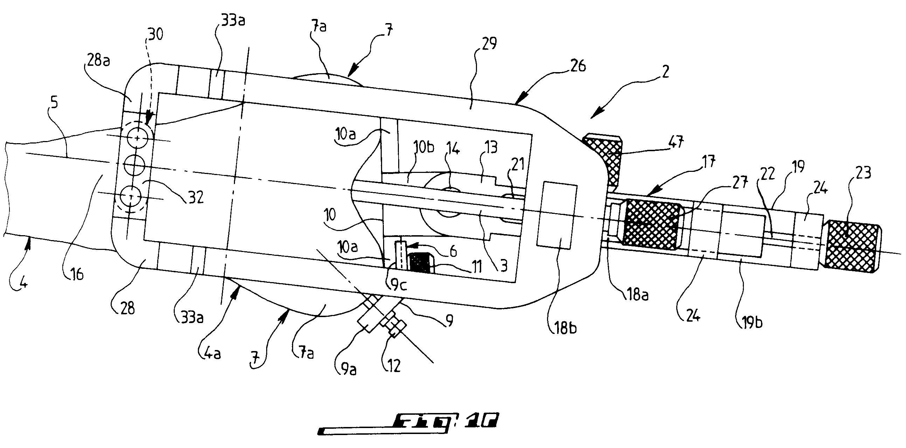

On va maintenant décrire le fonctionnement du dispositif ancillaire de l'invention en référence aux figures 1 et 5 à 12.

Après une préparation chirurgicale classique, le chirurgien vient forer, à partir du point d'entrée 45 (voir figures 2 et 5), la zone intercondylienne 39 de la trochlée pour reproduire l'axe anatomique du fémur, à l'aide d'un foret 46 (partiellement représenté).

Il introduit ensuite le guide intramédullaire 3 à partir du point d'entrée 45 dans le canal intramédullaire 45a, comme représenté sur la figure 6.

La tige du guide intramédullaire 3 présente une courbure 3b dont la convexité est tournée vers la face postérieure du fémur 4, pour dégager un espace suffisant pour permettre l'évolution de la rape 1 lorsqu'elle est guidée dans le logement incliné 35 du cadre 28.

On voit sur les figures 11 et 12 que le tronçon courbé 3b de la tige 3 est à l'aplomb du logement 35.

Le support 17 est ensuite mis en place sur l'extrémité 3a du guide intramédullaire 3, comme représenté sur la figure 9, et une mollette 47 vient immobiliser la tige 3 dans le corps principal 19b de l'équerre inférieure 19.

La plaque allongée 6 est plaquée contre les condyles 7 le long de la ligne bicondylienne 8 en faisant pivoter l'élément articulé 10 autour de l'axe 15 et coulisser le bras 13 dans l'extrémité inférieure 19a de l'équerre 19.

Il est également possible de régler la distance du support 17 par rapport aux condyles 7 en enfonçant plus ou moins la tige 3 dans le support 17.

Bien entendu, les différentes mollettes utilisées dans le dispositif ancillaire représenté ici, ainsi que les différentes liaisons glissières peuvent être remplacées par tout autre moyen équivalent.

Le chirurgien fait coulisser l'élément 9 sur une branche 10a de l'élément 10 jusqu'à ce que la bride d'extrémité longitudinale 9a vienne en regard d'un condyle 7, latéralement de manière que les clous 12 n'endommagent pas la zone de cartilage 48 de l'extrémité distale 4a du fémur 4.

A cet effet, les clous 12 et éventuellement la bride d'extrémité 9a sont prévus obliques par rapport à l'axe anatomique 5 de sorte que les clous 12 viennent s'implanter latéralement dans les condyles 7.

L'autre branche 10a de l'élément 10 est laissée libre pour ménager un espace suffisant pour les tendons de l'articulation.

Le chirurgien monte ensuite le guide de coupe 26 sur la branche verticale 18b du support 17 jusqu'à ce que le palpeur 30 vienne en butée contre la corticale antérieure 16 du fémur 4.

La dimension du palpeur 30 est déterminée de manière que la distance entre la surface de guidage principale 33a et la zone trochléenne à raper soit réglée automatiquement par aboutement du palpeur 30, la dimension du palpeur 30 étant adaptée au rayon de la rape 1 utilisée.

Dans la variante de réalisation représentée sur la figure 1, on a représenté une mollette 49 pour la fixation de l'étrier 29 sur le cadre 28, dans le cas où l'on veut rendre l'étrier amovible.

Avant de procéder à la coupe de la trochlée dans un plan tangent à la zone du cortex métaphysaire 16, on installe une paire de cales 50 (voir figure 11) dans les logements 35 en U incliné, de sorte que la face plane supérieure 50a des cales 50 définisse un plan unique avec la surface principale de guidage 33a des longerons 33.

Ces cales 50 permettent d'effectuer la coupe de la trochlée fémorale en deux étapes, d'abord suivant un plan tangent à la corticale antérieure 16, puis suivant un plan incliné parallèle à la zone intercondylienne 39 du fémur. Ces plans de coupe fictifs sont représentés en traits mixtes sur la figure 11.

On voit sur la figure 11 que la rape 1 est disposée au voisinage de l'extrémité avant 28a du cadre 28, l'arbre moteur 34 étant guidé par la surface principale 33a des longerons 33, l'étrier supérieur 29 servant de butée supérieure dans le cas d'un débattement vertical de l'arbre moteur 34. L'extrémité avant 28a présente une protubérance qui fait saillie vers le haut et sert de butée avant pour l'arbre moteur 34.

On voit sur les figures 1 et 10 que l'étrier 29 est ouvert à l'avant pour permettre l'installation et le retrait rapides de la rape 1 sur le cadre 28.

L'écartement entre les longerons 33 et l'étrier supérieur 29 peut être légèrement supérieur au diamètre de l'arbre moteur 34 pour permettre d'intercaler une bague en plastique 53 (voir figure 8).

Cette bague 53 est emmanchée sur l'arbre moteur 34 pour réduire les frottements et améliorer le calage de celui-ci dans le guide de coupe 26 par l'intermédiaire de la partie 53b de diamètre inférieur de la bague, la partie 53a de diamètre supérieur de la bague assurant un bon centrage de l'outil de coupe.

On notera que les extrémités libres 34a de l'arbre moteur 34 présentent une forme parallélipipédique pour leur liaison par exemple à un dispositif d'entraînement, non représenté.

L'étape de coupe illustrée sur la figure 11 forme la surface principale d'appui 37 et amène la face antérieure de l'épiphyse fémorale au même niveau que la corticale antérieure 16.

Le chirurgien enlève ensuite les cales 50 et introduit dans les logements 35 l'arbre de support 51a d'un gabarit cylindrique 51 illustré sur la figure 12 pour régler la position du guide de coupe 26 dans la direction de l'axe anatomique 5.

Le gabarit 51 présente une surface externe lisse destinée à venir s'appuyer contre la zone trochléenne inclinée 39.

Le rayon du gabarit 51 est égal au rayon de la rape 1 minoré de l'épaisseur maximale de la prothèse 42 à implanter la trochlée.

Ainsi, lorsque le gabarit 51 vient en butée contre l'extrémité distale 4a du fémur 4, en agissant sur la liaison glissière à vis 22, les logements 35 se trouvent à la distance adéquate du fémur pour effectuer la coupe suivant le plan fictif incliné qui est représenté en traits mixtes sur la figure 12.

Le chirurgien installe alors la rape 1 à l'entrée des logements 35 et effectue la coupe du fémur suivant ce plan incliné, comme représenté sur la figure 1.

Le chirurgien utilise enfin l'outil de coupe 52 illustré sur la figure 8, en forme de roue dentée, pour ménager la rainure 40 dans la surface d'appui inclinée 38 de l'empreinte trochléenne 36, après avoir enlevé l'étrier supérieur 29.

La roue dentée 52 peut comporter un diamètre supérieur à la rape 1 précitée et adapté à la profondeur de la rainure 40 à creuser, afin d'éviter l'utilisation d'un nouveau gabarit de perçage.

Les orifices 41 sont éventuellement réalisés avec un moyen de perçage classique.

Le chirurgien procède alors à la mise en place de la prothèse trochléenne 42 sur l'empreinte 36 en utilisant du ciment.

La stabilité de la prothèse 42 est assurée par la coupe très précise de l'empreinte trochléenne 36 et par la nervure 43 et éventuellement les ergots 41.

Bien que la présente invention ait été décrite en liaison avec des modes de réalisation particuliers, il est évident qu'elle n'y est nullement limitée et qu'on peut lui apporter diverses variantes et modifications sans pour autant sortir de son cadre ni de son esprit.

## Revendications

1. Dispositif ancillaire pour former une empreinte trochléenne (36) de réception d'une prothèse fémoro-patellaire (42), comportant :
- un guide d'alignement intramédullaire (3) à introduire dans l'extrémité distale du fémur suivant son axe anatomique (5),
- un support (17) monté, de préférence amovible, sur le guide intramédullaire et supportant des moyens de positionnement (6, 30) par rapport aux repères anatomiques du fémur, à savoir par exemple la ligne bicondylienne (8), l'extrémité distale des condyles (7) et la surface corticale antérieure (16),
- un guide de coupe (26) pour recevoir au moins un outil de coupe (1, 52),
caractérisé en ce que l'outil de coupe est un organe cylindrique dont la surface de révolution est apte à user ou à raper la zone trochléenne de l'extrémité fémorale (4a) sous l'action d'un arbre moteur (34) solidaire en rotation dudit organe (1), et que
- le guide de coupe précité (26) comporte des moyens de guidage en translation (33a, 35) de l'outil de coupe suivant deux plans sécants, l'un tangent à la corticale antérieure (16) et l'autre incliné sensiblement parallèlement à la zone intracondylienne (39).

2. Dispositif ancillaire selon la revendication 1, caractérisé en ce que le support (17) comporte des moyens pour déplacer le guide de coupe (26) et qui sont formés de deux liaisons glissières (18b, 22) orientées respectivement perpendiculairement à la corticale antérieure (16) et parallèlement au guide intramédullaire (3).

3. Dispositif ancillaire selon la revendication 1 ou 2, caractérisé en ce que les moyens de positionnement précités comprennent un moyen de butée formant palpeur (30) qui fait saillie vers le bas de l'extrémité libre avant (28a) du guide de coupe (26), la taille du palpeur (30) étant prédéterminée en fonction du rayon de l'outil de coupe utilisé pour régler automatiquement la distance séparant les moyens de guidage de la corticale antérieure, par aboutement du palpeur sur celle-ci.

4. Dispositif ancillaire selon l'une quelconque des revendications précédentes, caractérisé en ce qu'un tronçon (3b) du guide intramédullaire (3) est cintré et positionné entre l'extrémité fémorale distale (4a) et le support précité (17) pour laisser un espace suffisant pour permettre l'évolution de l'outil de coupe lorsque ce dernier effectue une coupe du fémur dans la zone intercondylienne inclinée (39).

5. Dispositif ancillaire selon l'une quelconque des revendications précédentes, caractérisé en ce que les moyens de positionnement comprennent en outre une plaque allongée (6) destinée à venir s'appuyer sur l'extrémité distale des condyles (7) le long de la ligne bicondylienne (8), et des clous de fixation (12) traversant une extrémité longitudinale (9a) de ladite plaque (6) pour venir se planter dans la zone des condyles non recouverte de cartilage (48), ladite plaque (6) étant constituée de deux éléments (9, 10) dont l'un (9) est coulissant par rapport à l'autre (10) dans la direction longitudinale de la plaque pour régler sa longueur en fonction de l'écartement des condyles (7), et ladite plaque (6) étant reliée audit support (17) par un bras articulé (13) autour d'un axe (15) sensiblement perpendiculaire à la corticale antérieure (16).

6. Dispositif ancillaire selon l'une quelconque des revendications précédentes, caractérisé en ce que les moyens de guidage se présentent sous la forme d'un cadre sensiblement rectangulaire (28) dont les deux longerons latéraux (33) ont pour fonction de supporter l'arbre moteur (34) de part et d'autre de l'organe cylindrique formant outil de coupe, chaque longeron (33) présentant une forme sensiblement en chevron qui définit une surface de guidage principale (33a) parallèle à la corticale antérieure (16) et se prolongeant par un logement de guidage (35) en forme de U incliné, dans lequel l'arbre moteur (34) est reçu pour réaliser la surface d'appui inclinée (38) dans le fémur (4).

7. Dispositif ancillaire selon la revendication 6, caractérisé en ce que le guide de coupe (26) comporte un étrier (29) surmontant le cadre précité (28), servant de butée supérieure dans le cas d'un débattement vertical de l'outil de coupe et ouvert vers l'avant pour permettre l'introduction et le retrait de l'outil de coupe dans le guide de coupe (26).

8. Dispositif ancillaire selon la revendication 6 ou 7, caractérisé en ce que le guide de coupe (26) comporte en outre une paire de cales (50) de forme complémentaire aux logements de guidage en U incliné et comportant une surface supérieure plane (50a) définissant un plan unique avec la surface de guidage principale (33a) des longerons (33) lorsque les cales (50) sont reçues dans les logements précités (35).

9. Dispositif ancillaire selon l'une quelconque des revendications précédentes, caractérisé en ce que l'outil de coupe est une rape (1) ou une roue dentée (52) dont les dents sont radialement inclinées pour venir creuser une rainure allongée (40) dans la trochlée fémorale.

10. Dispositif ancillaire selon l'une quelconque des revendications précédentes, caractérisé en ce qu'il comprend un gabarit de réglage (51) de la position du guide de coupe (26) à une distance adaptée du fémur (4) pour former la surface d'appui inclinée (38) de l'empreinte trochléenne (36), ce gabarit (51) présentant la forme d'un cylindre lisse dont le rayon est égal au rayon de l'organe cylindrique précité (1, 52) minoré de la profondeur maximale de l'empreinte (36) à former dans la trochlée.

11. Dispositif ancillaire selon l'une quelconque des revendications précédentes, caractérisé en ce qu'il comprend une bague (53) en plastique ou analogue formée d'une partie de diamètre supérieur (53a) et d'une partie de diamètre inférieur (53b) qui sont séparées par un épaulement radial, ladite bague étant destinée à s'emmancher sur l'arbre moteur (34) pour assurer un bon centrage de l'outil de coupe (1, 52) sur le guide de coupe (26) par l'intermédiaire de la partie de diamètre supérieur (53a), la partie de diamètre inférieur (53b) servant à caler l'arbre moteur (34) dans le guide de coupe (26).

12. Dispositif ancillaire selon la revendication 11, caractérisé en ce que la bague (53) appartient à une gamme de modules de dimensions prédéterminées en fonction des outils de coupe (1, 52) utilisés pour former différentes tailles d'empreinte trochléenne (36).

13. Prothèse fémoro-patellaire (42) présentant une face postérieure (42a) de forme adaptée à l'empreinte trochléenne (36) formée par le dispositif selon l'une quelconque des revendications précédentes et comportant une surface d'appui principale (37) plane et tangente à la corticale antérieure (16) et se prolongeant par une surface d'appui inclinée (38) sensiblement parallèle à la zone intracondylienne (39) de la trochlée fémorale, caractérisée en ce que la prothèse est limitée à la partie médiane et frontale de l'extrémité fémorale et présente des faces latérales (42b) destinées à venir se loger dans l'empreinte précitée pour préserver l'intégrité des condyles (7).

14. Prothèse fémoro-patellaire selon la revendication 13, caractérisée en ce qu'elle comprend une nervure allongée (43) et/ou au moins un ergot (44) en saillie de sa face postérieure (42a).
